(19) Europäisches Patentamt
European Patent Office
Office européen des brevets

(11) Veröffentlichungsnummer: **0 198 246**
**B1**

(12) # EUROPÄISCHE PATENTSCHRIFT

(45) Veröffentlichungstag der Patentschrift: **04.10.89**

(51) Int. Cl.⁴: **A61K 7/48**, C08B 37/08

(21) Anmeldenummer: **86103578.0**

(22) Anmeldetag: **17.03.86**

(54) **Kosmetische Mittel auf der Basis von quaternären hydroxyalkyl-substituierten Chitosanderivaten.**

(30) Priorität: **13.04.85 DE 3513277**

(43) Veröffentlichungstag der Anmeldung:
**22.10.86 Patentblatt 86/43**

(45) Bekanntmachung des Hinweises auf die Patenterteilung:
**04.10.89 Patentblatt 89/40**

(84) Benannte Vertragsstaaten:
**AT DE GB IT**

(56) Entgegenhaltungen:
**EP-A- 0 065 491**
**WO-A-84/02343**

**CHEMICAL ABSTRACTS, Band 46, Nr. 2, 25. Januar 1952, Seite 629a, Columbus, Ohio, US; S. HATTA et al.: "Macramin, a new high-molecular antibacterial substance derived from chitin"**
**CHEMICAL ABSTRACTS, Band 76, 1972, Seite 396, Nr. 46409n, Columbus, Ohio, US; M. TAKEDA: "Isomerization of D-glucose to D-fructose by a strongly basic polysaccharide 6-O-(2-hydroxyethyl) chitosan (trimethylammonium hydroxide)**

(73) Patentinhaber: **Wella Aktiengesellschaft, Berliner Allee 65, D-6100 Darmstadt(DE)**

(72) Erfinder: **Lang, Günther, Dr., Auf der Roten Erde 10 B, D-6107 Reinheim 5(DE)**
Erfinder: **Wendel, Harald, Grabengasse 3, D-6105 Ober-Ramstadt(DE)**
Erfinder: **Konrad, Eugen, Mecklenburger Strasse 101, D-6100 Darmstadt(DE)**
Erfinder: **Gross, Paul, Forstweg 54, D-6100 Darmstadt(DE)**
Erfinder: **Maresch, Gerhard, Höigesstrasse 19, D-6100 Darmstadt(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der Erfindung betrifft kosmetische Mittel zur Behandlung von Haaren oder der Haut mit einem Gehalt an makromolekularen quaternären, hydroxyalkyl-substituierten, vom Chitosan abgeleiteten Verbindungen in einer geeigneten Kosmetikgrundlage.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationaktiver Polymere in solchen kosmetischen Mitteln zahlreiche Vorteile ergibt; die Entwirrung der Haare sowie deren Behandlung wird erleichtert, und weiterhin wird dem Haar Sprungkraft und Glanzwirkung verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 2 627 419.

In gleicher Weise wie bei der Mehrzahl der kationaktiven Polymere mit quaternären Gruppierungen ergibt Chitosan ebenfalls häufig den Nachteil, daß es mit den anionaktiven oberflächenaktiven Agentien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampoonierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Aus der WO-A 8 402 343 sind quaternäre Chitosanderivate enthaltende kosmetische Mittel bekannt, die die vorstehend aufgeführten Nachteile nicht aufweisen. Diese Mittel besitzen jedoch, insbesondere wenn sie Parfümöle enthalten, nur eine begrenzte Lagerfähigkeit, was sehr leicht anhand der während der Lagerung auftretenden Veränderung von Farbe und Geruch dieser Mittel festgestellt werden kann. Aufgabe der Erfindung ist es, diese Nachteile zu beseitigen.

Es wurde nunmehr gefunden, daß kosmetische Mittel mit einem Gehalt an bestimmten quaternären Chitosanderivaten die vorstehend aufgeführten Nachteile nicht aufweisen.

Diese Mittel stellen kosmetische Mittel zur Behandlung von Haaren oder der Haut dar, die sich durch überraschend vorteilhafte Eigenschaften auszeichnen und die dadurch gekennzeichnet sind, daß sie in einer geeigneten Kosmetikgrundlage eine quaternäre makromolekulare, vom Chitosan abgeleitete polymere Verbindung der allgemeinen Formel I

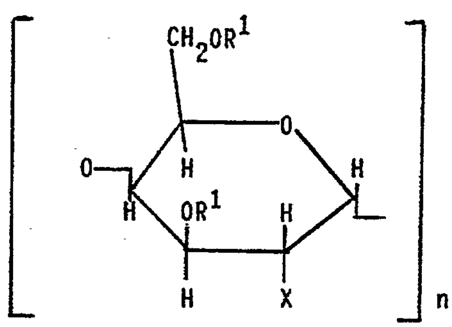

(I),

wobei X = die Bedeutung $X_1$, $X_2$ oder $X_3$, mit

$X_1$ = 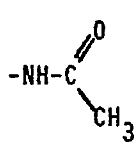

$X_2 = -N(R^2)_3{}^+ Y^-$ (Y = Cl,Br,J,$HSO_4$ oder $CH_3SO_4$)

$X_3 = -N(R^2)_mH_{(2-m)}$ (m=0,1 oder 2)

hat und die Anteile der Substituenten $X_1$, $X_2$ und $X_3$, bezogen auf die Gesamtzahl der Monomereinheiten,

$X_1$ = 0 - 50%, $X_2$ = 1 - 100% und $X_3$ = (100 - $X_1$ - $X_2$) % betragen, n eine ganze Zahl von 1002 bis 2002 bedeutet,

$$R^1 = H, \ -CH_2CH_2-OH, \ -CH_2-\underset{\displaystyle OH}{\underset{|}{C}H}-CH_3$$

bedeutet und
$R^2$ = $-CH_3$ oder $-CH_2-CH_3$ darstellt,
enthalten.

Die Herstellung der Verbindung der Formel I ist bekannt und unter anderem in der Literatur I.E.Malz EP-OS 0065 491, S.Okimasu, Bull.Agr.Chem.Soc.Jap. 20, 29 (1956) beschrieben. Quaternäre O-hydroxypropylierte Verbindungen der Formel I können ebenfalls durch N-Alkylierung nach S.Okimasu aus dem in der japanischen offengelegten Patentanmeldung 180 602 von 1982 beschriebenen O-hydroxypropylierten Chitosan oder nach dem nachfolgenden Herstellungsbeispiel 13 erhalten werden.

Die hier beschriebenen Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionenen, Fönlotionen, Mittel zur Festigung der Frisur, Waschlotionen, Haarkuren, Mittel gegen Kopfschuppen, Mittel zur permanenten Haarverformung, Mittel zur Färbung oder Entfärbung von Haaren, Mittel zum Auftragen vor oder nach dr Haarfärbung und als kosmetische Mittel zur Pflege, zum Schutz oder zur Reinigung der Haut wie Gesichtswässer, Rasierwässer, Feuchthaltecremes, Coldcremes, Körperlotionen, Sonnenschutzmittel oder auch Make-up-Präparate wie Schminkcremes und Rouges.

Der Gehalt der erfindungsgemäß kosmetischen Mittel an den Chitosanderivaten der Formel I liegt zweckmäßig bei 0,05 bis 10 Gew. %, vorzugsweise bei 0,05 bis 3,0 Gew. %.

Die kosmetischen Mittel gemäß der vorliegenden Erfindung können zusätzlich zu dem Chitosanderivat der Formel I zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemäßen kosmetischen Mittel weisen in bevorzugter Weise einen pH-Wert von 2 bis 11 auf und können in Form wäßriger, alkoholischer oder wäßrig-alkoholischer Zubereitungen, z.B. mit einem Alkohol mit 1 bis 4 Kohlenstoffatomen, als Lösungen, als Cremes, als Gele, als Dispersionen oder als Emulsionen vorliegen. Ebenfalls ist es möglich, diese Mittel mit Hilfe eines Zerstäubers bze. anderer geeigneter Sprühvorrichtungen oder im Gemisch mit üblichen Treibgasen als Aerosolspray aus einem Druckbehälter zu versprühen.

Wenn es sich bei den erfindungsgemäßen kosmetischen Mitteln um Mittel zur Festigung der Frisur, wie flüssige Haarfestiger oder Haarsprays, handelt, dann liegen sie üblicherweise als wäßrige oder wäßrig-alkoholische Lösungen vor, die durch einen Gehalt an quaternären Chitosanderivaten der vorstehend genannten Formel I gekennzeichnet sind. Hierbei können die quarternären Chitosanderivate selbst als filmbildenes bzw. festigendes Harz eingesetzt werden. Es können jedoch auch zusätzlich andere filmbildende natürliche oder synthetische Polymere in dem erfindungsgemäßen Haarfestigungsmittel enthalten sein. Als natürliche Polymere kommen beispielsweise Schellack,Alginate, Gelatine, Pektine und Cellulosederivate in Betracht. Von den synthetischen Polymeren finden z.B. Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen bzw. die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung. Die Mittel weisen dan insbesondere einen pH-Wert zwischen 6 und 8 auf. Solche Mittel zur Festigung der Frisur enthalten üblicherweise filmbildende Polymere in einer Gesamtmenge von 0,05 bis 3,0 Gew. %. Enthalten die Mittel neben den quaternären Chitosanderivaten der Formel I noch andere filmbildende Polymere, so reduziert sich der Gehalt an quaternären Chitosanderivaten entsprechend.

Als Alkohole kommen insbesondere die für kosmetische Zwecke üblicherweise verwendeten niederen Alkohole wie Ethanol und Isopropanol in Betracht.

Die erfindungsgemäßen Mittel zur Festigung der Frisur können weiterhin die üblichen Zusätze wie beispielsweise Parfümöl, Bakterizide oder Fungizide, kämmbarkeitsverbessernde Substanzen usw. enthalten.

Die erfindungsgemäßen Mittel zur Festigung der Frisur können gegebenenfalls durch einem Gehalt an kosmetischen Farbstoffen das Haar gleichzeitig färben oder tönen. Derartige Präparate sind u.a. als Farbfestiger oder Tönungsfestiger im Handel bekannt. Sie enthalten zusätzlich übliche für Haarfestiger bekannte Farbstoffe wie biespielsweise aromatische Nitrofarbstoffe (z.B. 1,4-Diamino-2-nitrobenzol), Azofarbstoffe (z.B. C.I. Acid Brown 4), Anthrachinonfarbstoffe (z.B. C.I. Disperse Violet 4) und Triphenylmethanfarbstoffe (z.B. C.I. Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise 0,01 bis 2,0 Gew. %.

Die erfindungsgemäßen Mittel zur Festigung der Frisur weisen bei gleich guter Festigung des Haares gegenüber üblichen Mitteln eine verbesserte Substantivität zum Haar, eine besonders gute Kämmerbarkeit und einen guten Griff des Haares im nassen Zustand sowie einen besonders angenehmen Griff des Haares im getrockneten Zustand auf.

Wenn die erfindungsgemäßen Mittel Haarwaschmittel darstellen, liegen sie in Form wäßriger Lösungen oder Emulsionen vor und enthalten neben dem Chitosanderivat zumindest ein anionisches, kationisches, nichtionisches oder amphoteres Tensid.

In diesem Haarwaschmittel liegt die Konzentration des Tensides im allgemeinen zwischen 3 und 50 Gew. % und vorzugsweise zwischen 3 und 25 Gew. %, bezogen auf das Gesamtgewicht des Mittels, wobei der pH-Wert im allgemeinen zwischen 3 und 9 und vorzugsweise zwischen 4 und 7 liegt.

Der erfindungsgemäßen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Copra-Fettsäuren, Lauryl- oder Ölsäuremono- oder -diethanolamid, die zweckmäßigerweise in Mengen von 1 bis 10 und vorzugsweise 1 bis 3 Gew. %, bezogen auf das Gesamtgewicht des Mittels Verwendung finden, angeführt werden.

Unter den Verdickern können insbesondere die Acrylpolymere und die Cellulosederivate wie Carboxymethylcellulose, Hydroxypropylmethylcellulose, Hydroxyethylcellulose angeführt werden. Die Verdicker liegen im allgemeinen in einem Anteil von 0,1 bis 5 Gew. % vor.

Unter den Tensiden oder oberflächenaktiven Agentien, die in Kombination mit den quaternären Chitosanderivaten verwendet werden, können beispielsweise die folgenden genannt werden:

a) die anionischen oberflächenaktiven Agentien, wie beispielsweise die Alkali-, oder Erdalkali-, Ammonium- oder Alkanolaminsalze von Alkansulfonaten, Alkylsulfaten und Alkylethersulfaten, die $C_{12}$-$C_{18}$-Alkyl- und insbesondere $C_{12}$-$C_{14}$-Alkyl-Sulfatnatriumsalze oder Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agentien, wie beispielsweise das oxethylierte Fettalkohole mit 12 bis 18 Kohlenstoffatomen, z.B., mit bis zu 40 Mol Ethylenoxid pro Mol Fettalkohol, oxethylierter Laurin-, Tetradecyl-, Cetyl-, Olein-, Palmitin-, und Stearin-alkohol, allein oder im Gemisch, die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül sowie Fettsäurealkanolamide;

c) die kationischen oberflächenaktiven Agentien, wie beispielsweise das Dilauryldimethylammoniumchlorid, die Chloride oder Bromide von Alkyldimethylbenzylammonium, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, Dialkyldimethylammoniumchloride oder -bromide, Alkylpyridiniumsalze, beispielsweise Cetylpyridinumchlorid, Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agentien wie beispielsweise die Carboxylderivate von Imidazol, die N-Alkylbetaine, die N-Alkylsulfobetaine, die N-Alkylaminobetaine, die N-Alkylaminopropionate, die Alkyldimethylammoniumacetate bzw. die $C_{12}$-$C_{18}$-Alkyldimethylcarboxymethylammoniumsalze.

Die erfindungsgemäßen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder Suspensionen vor und enthalten zusätzlich zu den Chitosanderivaten der Formel I kationische, nichteionogene, amphotere oder anionische Emulgatoren sowie als Bestandteil der Ölphase z.B. Fettalkohole, Fettsäure-ester oder -amide, weiterhin Parfümöle, Vaseline, Wollfettalkohol oder feste oder flüssige Paraffine.

Wenn die erfindungsgemäsen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe, beispielsweise aus der Gruppe der aromatischen Diamine bzw. Aminophenole. Weiterhin können diese Mittel gegebenfalls Alkalisierungsmittel, Antioxidantien sowie weitere für solche Mittel übliche kosmetische Zusätze und Hilfsstoffe enthalten.

Die erfindungsgemäßen Mittel können auch Dauerverformungsmittel oder Fixiermittel für Haare darstellen. Sie enthalten dann zusätzlich zu den genannten Chitosanderivaten der Formel I Reduktionsmittel, wie zum Beispiel Thioglykolsäure,

Thiomilchsäure und Ammoniumsulfit bzw. Oxidationsmittel, wie zum Beispiel Wasserstoffperoxid oder Natriumbromat sowie gegenbenenfalls Alkalisierungsagentien bzw. Peroxidstabilisatoren, z.B. Phosphorsäure, ferner andere kosmetische Hilfsstoffe und Zusatzstoffe wie beispielsweise Parfümöle, Riechstoffe, Pflegestoffe und Farbstoffe.

Wie bereits erwähnt wurde, können die erfindungsgemäßen kosmetischen Mittel auch zur Behandlung der Haut verwendet werden.

In der Tat erleichtern diese kosmetischen Mittel die Befeuchtung der Haut und verhindern das Austrocknen. Diese Mittel verleihen der Haut weiterhin eine hervorragende Weichheit im Griff.

Die erfindungsgemäßen kosmetischen Mittel liegen hierzu vorzugsweise in Form vom Cremes, Gelen, Emulsionen oder wäßrigen, alkoholischen oder wäßrig-alkoholischen Lösungen vor, die das Chitosanderivat der Formel I in einer Konzentration von 0,1 bis 10 Gew. % und vorzugsweise von 0,2 bis 6 Gew. % enthalten.

Die im allgemeinen in diesen Kosmetikzubereitungen enthaltenen Hilfsstoffe sind beispielsweise Duftstoffe, Farbstoffe, Konservierungsmittel, Verdickungsmittel, Sequestriermittel, Emulgiermittel, Sonnenschutzfilter etc..

Diese Zubereitungen für die Hautpflege liegen insbesondere in Form von Cremes oder Lotionen zur Pflege der Hände oder des Gesichts oder in Form von Sonnenschutzcremes, gefärbten Cremes, Abschminkmilchprodukten, Schaumbad- und Duschbad-Präparaten oder auch in Form von Deodorierzubereitungen vor.

Diese Zubereitungen werden unter Anwendung klassischer Verfahrensweisen hergestellt.

Beispielsweise kann man zur Bildung einer Creme eine wäßrige Phase, die das erfindungsgemäße Chitosanderivat und gegebenenfalls andere Bestandteile oder Hilfsstoffe gelöst enthält, und eine ölige Phase emulgieren.

Für die ölige Phase kann man verschiedenartige Verbindungen verwenden, beispielsweise Paraffinöl, Vaselinöl, Süßmandelöl, Avocadoöl, Olivenöl, Fettsäureester, wie Glycerylmonostearat, Ethylpalmitat oder Isopropylpalmitat oder Alkylmyristate, wie Propylmyristat, Butylmyristat oder Cetylmyristat. Man kann sie auch mit Fettsäurealkoholen, wie Cetylakohol oder Wachsen, beispielsweise Bienenwachs, versetzen.

Die Chitosanderivate der Formel I können in den Kosmetikzubereitungen für die Hautpflege entweder als Hilfsstoff oder als Hauptwirkstoff enthalten sein.

Die nachfolgenden Beispiele sollen den Gegenstand der Erfindung näher erläutern.

Beispiel 1

| Haarfestiger | |
|---|---|
| 0,6 g | O-Hydroxyethyl-N-trimethyl-Chitosan-Jodsalz $(C_{11}H_{22}O_5NJ)_n$; Substitutionsgrad: trimethyl = 0,93, hydroxyethyl = 1,1. |
| 73,8 g | Wasser |
| 25,0 g | Isopropanol |
| 0,4 g | 10%ige Ameisensäure |
| 0,2 g | Parfümöl |
| 100,0 g | |

20 ml dieser Lösung wurden auf gewaschene, handtuchtrokkene Haare verteilt, das Haar in üblicher Weise zur Frisur eingelegt und getrocknet. Bei guter Festigungswirkung zeigte das Haare, im Vergleich zu einem Haarfestiger auf der Basis von Chitosan/Ameisensäure, einen angenehmeren und weicheren Griff.

Beispiel 2

| Tönungsfestiger | |
|---|---|
| 1,00 g | N-Triethyl-Chitosan nach Beispiel 2 der EP-OS 0 065 491 (Substitutionsgrad Triethyl = 0,84) |
| 1,00 g | Milchsäure |
| 0,10 g | Cetyltrimethylammoniumchlorid, 50%ige wäßrige Lösung |
| 0,05 g | Acid Brown 4 (C. I. 41 805) |
| 97,85 g | Wasser |
| 100,00 g | |

20 ml dieser Lösung wurden auf dem gewaschenen, handtuchtrockenen Haar verteilt und das Haar in üblicher Weise eingelegt und getrocknet. Das Haar zeigte anschließend eine leichte Rot-Braunfärbung.

Beispiel 3

| Tönungsfestiger | |
|---|---|
| 0,60 g | quaternäres Chitosanderivat nach Beispiel 2 |
| 0,15 g | 1,4-Di(β-hydroxyethylamino)-2-nitro-5-chlorbenzol |
| 25,00 g | Ethanol |
| 74,25 g | Wasser |
| 100,00 g | |

20 ml dieser Lösung wurden auf die gewaschenen, handtucktrockenen Haar gegeben, sodann das Haar eingelegt und getrocknet. Die Haare waren rot-violett gefärbt und gefestigt.

Beispiel 4

| Anionisches Haarwaschmittel | |
|---|---|
| 1,00 g | quaternäres Chitosanderivat nach Beispiel 13 |
| 40,00 g | Laurylalkoholdiglykolethersulfat-Natriumsalz, 28%ige wäßrige Lösung |
| 4,00 g | Natriumchlorid |
| 0,05 g | Farbstoff |
| 54,85 g | Wasser |
| 0,10 g | Formaldehyd, 25%ige wäßrige Lösung |
| 100,00 g | |

Es wurde ein klares Shampoo erhalten. Das damit gewaschene Haar war hinsichtlich Griff, Glanz und Kämmbarkeit ausgezeichnet konditioniert. Infolge der Verträglichkeit des quaternären Chitosanderivates mit Alkylethersulfat kann das vorstehend aufgeführte Shampoo erstellt werden, mit dem eine gleichzeitige Reinigung und Pflege des Haares möglich ist.

Beispiel 5

| Amphoteres, tönendes Haarwaschmittel | |
|---|---|
| 2,00 g | quaternäres Chitosanderivat nach Beispiel 1 |
| 40,00 g | dimethyl-carboxymethylen-propylenamido-stearat-betain, 35%ige wäßrige Lösung |
| 5,06 g | Ameisensäure, 10%ig |
| 3,50 g | Kokosfettsäurediethanolamid |
| 1,00 g | Pikraminsäure (C.I. 76 540), 1%ige wäßrige Lösung |
| 48,44 g | Wasser, vollentsalzt |
| 100,00 g | |

Das Haar wurde mit etwa 15 bis 20 g des obigen Mittels einshampooniert. Nach einer Einwirkungszeit von 5 bis 10 Minuten spülte man mit Wasser aus. Das Haar war gelborange getönt und ausgezeichnet konditioniert, besonders hinsichtlich Griff und Kämmbarkeit.

Beispiel 6

| Haarkurmittel, kationisch | |
|---|---|
| 0,30 g | quaternäres Chitosanderivat nach Beispiel 2 |
| 4,00 g | Cetylstearylalkohol |
| 1,48 g | Milchsäure, 10%ig |
| 2,50 g | Kokos(pentaethoxy)methylammoniumchlorid |
| 1,00 g | Sorbitanmonopalmitat mit 20 Mol Ethylenoxid |
| 90,72 g | Wasser, vollentsalzt |
| 100,00 g | |

Beispiel 7

| Haarkurmittel, gelförmig | |
|---|---|
| 2,10 g | quaternäres Chitosanderivat nach Beispiel 2 |
| 0,60 g | Hydroxypropylmethylcellulose |
| 0,50 g | Laurylpyridiniumchlorid |
| 96,80 g | Wasser, vollentsalzt |
| 100,00 g | (auf pH 5,0 mit 10%iger Ameisensäure eingestellt) |

Jeweils 35 g der Haarkurmittel nach Beispiel 9 bzw. 10 wurden im gewaschenen Haar verteilt und nach einer Einwirkungszeit von 3 bis 5 Minuten mit Wasser wieder aus. gespült; als Ergebnis wurde ein ausgezeichneter Griff, Glanz sowie Kämmbarkeit des Haares festgestellt.

Beispiel 8

| Hautcreme | |
|---|---|
| 0,30 g | quaternäres Chitosanderivat nach Beispiel 1 |
| 3,00 g | Stearylalkohol |
| 1,00 g | Wollfettalkohol (Adeps Lanae) |
| 1,00 g | Vaseline |
| 0,76 g | Milchsäure, 10%ig |
| 1,00 g | Natriumcetylstearylsulfat |
| 92,94 g | Wasser, vollentsalzt |
| 100,00 g | |

Beispiel 9

| Haartönungsmittel | |
|---|---|
| 0,50 g | quaternäres Chitosanderivat nach Beispiel 1 |
| 12,00 g | Cetylstearylalkohol |
| 0,10 g | Parahydroxybenzoesäureethylester |
| 6,00 g | Laurylalkohol-diglycolethersulfat-Natriumsalz (28%ige wäßrige Lösung) |
| 0,50 g | Parfümöl |
| 79,31 g | Wasser |
| 0,50 g | 1-Hydroxy-2-amino-4-nitrobenzol (C.I. 76 530) |
| 0,85 g | 1,4-Diamino-2-nitrobenzol (C.I. 76 070) |
| 0,24 g | Natriumhydroxyd |
| 100,00 g | |

Ungefähr 30 bis 40 g wurden in dem gewaschenen Haar verteilt und nach einer Einwirkungszeit von 20 Minuten ausgespült. Das Haar war rötlich gefärbt und wies eine gute Kämmbarkeit und einen angenehmen Griff auf.

Beispiel 10

| Oxidationshaarfärbemittel | |
|---|---|
| 0,50 g | quaternäres Chitosanderivat nach Beispiel 2 |
| 0,08 g | 3,5-Diamino-2,6-dimethoxypyridin-dihydrochlorid |
| 0,30 g | 1,4-Diaminobenzol |
| 0,25 g | Resorcin |
| 0,30 g | Natriumsulfit |
| 3,50 g | Laurylalkohol-diglycolethersulfat-Natriumsalz (28%ige wäßrige Lösung) |
| 15,00 g | Cetylalkohol |
| 3,00 g | Ammoniak |
| 77,07 g | Wasser |
| 100,00 g | |

50 g dieses Haarfärbemittels wurden mit 50 ml 6 %-iger Wasserstoffperoxidlösung gemischt und auf weißes Haar aufgetragen. Nach 30 Minuten wurde das Haar mit Wasser ausgespült und getrocknet. Das Haar hatte eine natürlich wirkende matt-blonde Färbung sowie einen natürlichen angenehmen Griff.

Beispiel 11

| Dauerwellmittel | |
|---|---|
| 0,50 g | quaternäres Chitosanderivat nach Beispiel 1 |
| 10,00 g | Thioglykolsäure |
| 8,00 g | Ammoniak, 25%ig |
| 6,10 g | Ammoniumhydrogencarbonat |
| 75,40 g | Wasser |
| 100,00 g | |

Zur Anwendung trug man dieses Dauerwellmittel auf deas gewickelte handtuchtrockene Haar gleichmäßig auf und ließ es etwa 20 Minuten einwirken; danach wurde das Haar mit Wasser ausgespült und in bekannter Weise oxidativ behandelt. Es wurde eine gutes Wellergebnis erhalten, die Haare fühlten sich natürlich und weich an.

Beispiel 12

| Haarfestiger Alkoholfrei | |
|---|---|
| 0,70 g | quaternäres Chitosanderivat nach Beispiel 2 |
| 1,50 g | Ameisensäure 10%ig |
| 0,80 g | Parfüm |
| 0,10 g | Chloracetamid (Konservierungsmittel) |
| 96,90 g | Wasser, vollentsalzt |
| 100,00 g | |

Beispiel 13

Herstellung von N-Trimethyl-O-hydroxypropylchitosanmethylsulfat

16,1g≙0,1 Mol niedermolekulares Chitosan werden mit 325 ml 43 %iger NaOH versetzt und nach der von DANILOV, S.N. und PLISKO, E.A. (Zhur. Obs. Khim 28, 2255-59 (1958) ) beschriebenen Methode (3 x 30 minütiges Abkühlen auf - 78°C, 2 x 60 minütiges Auftauen bei Raumtemperatur gefolgt vom Auftauen über Nacht) in Alkalichitosan umgewandelt. Anschließend saugt man auf ein Feuchtgesamtgewicht von etwa 160 g ab, trägt das Alkalichitosan in 200 - 300 ml Propylenoxid ein und rührt die Dispersion insgesamt 24 h lang bei 35°C.

Nach beendeter Reaktion trennt man durch Filtration, wäscht den Filterrückstand mit organischen Lösungsmitteln (Isopropanol, Aceton) alkylierungsmittelfrei, dispergiert in Wasser, stellt die Dispersion neutral ein und schließt ein Reinigung des Produktes durch Dialyse an.

Das erhaltene O-Hydroxypropylchitosan besitzt folgende Kenngrößen:

Grenzviskositätszahl [η] in 0,2 M $CH_3$-COOH + 0,1 M $CH_3COONa$ bei 25°C = 137 ml•g$^{-1}$
Wasserdampfaufnahme zwischen 30 und 70% relativer Luftfeuchte = 13,9 Gew.%; Hydroxypropyl - Substitutionsgrad errechnet aus NMR-Daten = 1,53.

Zu der klaren, viskosen Lösung von 14,27 g≙ 0,06 Mol des O-Hydroxy-propylchitosan-Zwischenproduktes in 100 ml Wasser und 15 ml Dioxan fügt man 30,24 g ≙ 0,36 Mol $NaHCO_3$ hinzu, wobei eine leichte Trübung auftritt.

Nach dem Erwärmen dieses Gemisches auf ca. 40°C tropft man unter intensivem Rühren 26 ml≙ 34,55 g≙ 0,27 Mol Dimethylsulfat zu und verkocht nach Beendigung der $CO_2$- Entwicklung das überschüssige Alkylierungsmittel durch einstündiges Erwärmen auf 100°C. Es resultiert eine klare Lösung, die einer 8-tägigen Dialyse unterworfen wird. Die dialysierte, druckfiltrierte Lösung wird eingeengt und in Aceton ausgefällt. Der flockige Niederschlag wird sodann gesammelt und bei 50°C im Vakuumtrockenschrank getrocknet.

Man erhält 12,2 g N-quaternäres, O-hydroxyalkyliertes Chitosanderivat mit den nachstehenden Kenndaten:

Substitutionsgrad Hydroxypropyl: 1,25 } aus NMR-Daten
Substitutionsgrad Trimethyl: 0,72 }

Grenzviskositätszahl [η] in 0,2M $CH_3COOH$ + 0,1M NaCl bei 25°C = 60[ml•g$^{-1}$]

## Patentansprüche

1. Kosmetisches Mittel zur Behandlung von Haaren oder der Haut, dadurch gekennzeichnet, daß es in einer geeigneten Kosmetikgrundlage eine quaternäre makromolekulare vom Chitosan abgeleitete polymere Verbindung der allgemeinen Formel I

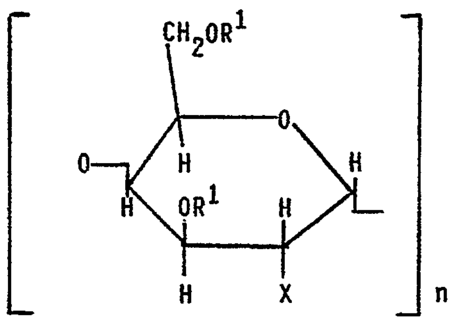

(I),

wobei
X die Bedeutung $X_1$, $X_2$ oder $X_3$, mit

$X_1$ = 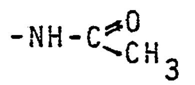

$X_2 = -N(R^2)_3^+ Y^-$ (Y = Cl,Br,J,$HSO_4$ oder $CH_3SO_4$)
$X_3 = -N(R^2)_mH_{(2-m)}$ (m = 0, 1 oder 2)
hat, und die Anteile der Substituenten $X_1$, $X_2$ und $X_3$, bezogen auf die Gesamtzahl der Monomereinheiten,
$X_1$ = 0 - 50 %, $X_2$ = 1 - 100 % und
$X_3$ = (100 = $X_1$ - $X_2$) % betragen, n eine ganze Zahl von 1002 bis 2002 bedeutet,

$R^1 = H, CH_2CH_2OH$ oder $CH_2-\underset{OH}{\underset{|}{CH}}-CH_3$ bedeuten und

$R^2 = CH_3$ oder $CH_2CH_3$ darstellt,
enthält.

2. Mittel nach Anspruch 1, dadurch gekennzeichnet, daß die Verbindung der Formel I ein O-Hydroxyethyl-N-trimethyl-Chitosansalz oder ein O-Hydrodypropyl-N-trimethyl-Chitosansalz ist.

3. Mittel nach Anspruch 1, dadurch gekennzeichnet, daß die Verbindung der Formel I ein N-Triethyl-Chitosansalz ist.

4. Mittel nach Anspruch 1 bis 3, dadurch gekennzeichnet, daß es die polymere Verbindung der Formel I in einer Menge von 0,05 bis 10,0 Gew.-% enthält.

5. Mittel nach Anspruch 1 bis 4, dadurch gekennzeichnet, daß es als Kosmetikgrundlage eine wäßrige, alkoholische oder wäßrig-alkoholische Lösung, eine Creme, eine Gel oder eine Emulsion enthält.

6. Mittel nach Anspruch 1 bis 5, dadurch gekennzeichnet, daß es als Kosmetikgrundlage eine wäßrige oder wäßrig-alkoholische Lösung eines niedermolekularen Alkohols, wie Ethanol oder Isopropanol, umfaßt und einen pH-Wert zwischen 6 und 8 aufweist.

7. Mittel nach Anspruch 1 bis 5 dadurch gekennzeichnet, daß es zusätzlich ein kationisches, nichtionisches, amphoteres oder anionisches Tensid enthält und in Form eines Haarwaschmittels vorliegt.

8. Mittel nach Anspruch 7, dadurch gekennzeichnet, daß das anionische Tensid ein Alkylethersulfat ist.

9. Mittel nach Anspruch 7 und 8, dadurch gekennzeichnet, daß es das Tensid in einer Konzentration zwischen 3 und 25 Gew.-% enthält und einen pH-Wert zwischen 4 und 7 aufweist.

10. Mittel nach Anspruch 1 bis 6, dadurch gekennzeichnet, daß es als Kosmetikgrundlage eine alkoholische oder wäßrig-alkoholische Lösung enthält, die im Gemisch mit einem üblichen Treibgas in einem Druckbehälter abgefüllt ist und in Form eines Aerosolsprays vorliegt.

11. Mittel nach Anspruch 1 bis 3, dadurch gekennzeichnet, daß die Kosmetikgrundlage eine wäßrige, alkoholische oder wäßrig-alkoholische Lösung, eine Creme, ein Gel oder eine Emulstion ist, in der das Chitosanderivat der Formel I in einer Konzentration zwischen 0,1 und 10 Gew.-% enthalten ist, und es in Form eines Hautbehandlungsmittels vorliegt.

12. Mittel nach Anspruch 1 bis 10, dadurch gekennzeichnet, daß es zusätzlich ein bekanntes filmbildendes synthetisches oder natürliches kosmetiches Polymer enthält.

## Claims

1. Cosmetic composition for the treatment of hair or skin, characterized in that it contains in a suitable cosmetic base a quaternary macromolecular polymeric compound derived from chitosan and having the general formula (I)

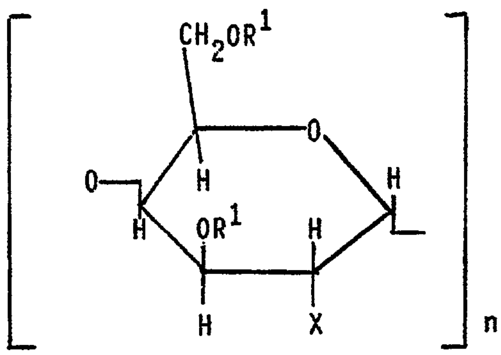 (I),

wherein
X designates $X_1$, $X_2$ or $X_3$, with

$X_1$ = 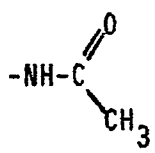

$X_2 = -N(R^2)_3^+Y^-$ (Y = Cl, Br, J, $HSO_4$ or $CH_3SO_4$)
$X_3 = -PN(R^2)_mH_{(2-m)}$ (m = 0, 1 or 2)
and the proportions of substituents $X_1$, $X_2$ und $X_3$, with reference to the total number of the monomer units, amount to
$X_1$ = 0–50%, $X_2$ = 1–100% and
$X_3$ = (100–$X_1$–$X_2$)%,
n being an integer from 1002 to 2002,

$R^1$ = H. $CH_2-CH_2-OH$ or $CH_2-\underset{OH}{CH}-CH_3$ and

$R^2$ = $CH_3$ or $CH_2-CH_3$.

2. Composition according to claim 1, characterized in that said compound of formula (I) is a O-hydroxyethyl-N-trimethyl-chitosan salt or a O-hydroxypropyl-N-trimethyl-chitosan salt.

3. Composition according to claim 1, characterized in that said compound of formula (I) is a N-triethylchitosan salt.

4. Composition according to claim 1 to 3, characterized in that it contains said polymeric compound of formula (I) in a quantity of 0.05 to 10 percent by weight.

5. Composition according to claim 1 to 4, characterized in that it contains as cosmetic base an aqueous, alcoholic or aqueous-alcoholic solution, a cream, a gel or an emulsion.

6. Composition according to claim 1 to 5, characterized in that it contains as a cosmetic base an aqueous or an aqueous-alcoholic solution of a low molecular alcohol, such as ethanol or isopropanol, and comprises a pH-value between 6 and 8.

7. Composition according to claim 1 to 5, characterized in that it contains, in addition, a cationic, nonionic, amphoteric or anionic surfactant and is in the form of a hair shampoo.

8. Composition according to claim 7, characterized in that said anionic surfactant is an alkylethersulfate.

9. Composition according to claim 7 and 8, characterized in that it contains said surfactant in a concentration between 3 and 25 percent by weight and has a pH-value between 4 and 7.

10. Composition according to claim 1 to 6, characterized in that it contains as cosmetic base an alcoholic or aqueous-alcoholic solution which is filled into a pressurized container in admixture with a conventional gas and is in the form of an aerosol spray.

11. Composition according to claim 1 to 3, characterized in that said cosmetic base is an aqueous, alcoholic or aqueous-alcoholic solution, a cream, a gel or an emulsion in which said chitosan derivative of formula (I) is contained in a concentration between 0.1 to 10 percent by weight and it is in the form of a skin treatment composition.

12. Composition according to claim 1 to 10, characterized in that it contains, in addition, a known film forming synthetic or natural cosmetic polymer.

## Revendications

1. Agent cosmétique pour le traitement des cheveux ou de la peau, caractérisé en ce qu'il contient dans une base cosmétique appropriée, un composé polymère macromoléculaire quaternaire derivée du chitosane, de formule générale (I)

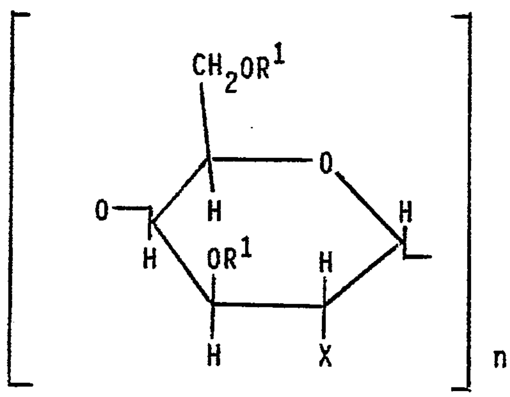

(I),

où X représente $X_1$, $X_2$ ou $X_3$, avec

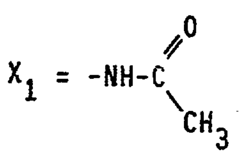

$X_2 = -N(R^2)_3^+Y^-$ (Y = Cl, Br, J, $HSO_4$ ou $CH_3SO_4$)
$X_3 = -N(R^2)_mH_{(2-m)}$ (m = 0, 1 ou 2)
et les parts des groupes $X_1$, $X_2$ und $X_3$, rapportée à la nobre totale d'unités monomères, font $X_1$ = 0 – 50%, $X_2$ = 1 – 100 % et $X_3$ = (100 = $X_1$ – $X_2$) % n représente un nombre entier de 1002 à 2002.

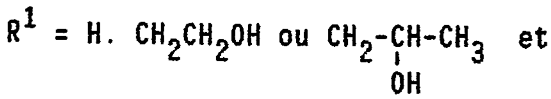

$R_2 = CH_3$ ou $CH_2CH_3$.

2. Agent selon la revendication 1, caractérisé en ce que le composé de formule (I) est un sel du O-hydroxyethyl-N-trimethyl-chitosane ou un sel du O-hydroxypropyl-N-trimethyl-chitosane.

3. Agent selon la revendication 1, caractérisé en ce que le composé de formule (I) est un sel du N-triethyl-chitosane.

4. Agent selon les revendications 1 à 3, caractérisé en ce qu'il contient le composé polymère de formule (I) à raison de 0,05 à 10% en poids.

5. Agent selon les revendications 1 à 4, caractérisé en ce qu'il contient comme base cosmétique une solution aqueuse, alcoolique ou hydro-alcoolique, une crème, un gel ou une emulsion.

6. Agent selon les revendications 1 à 5, caractérisé en ce qu'il contient comme base cosmétique une solution aqueuse ou hydro-alcoolique d'un alcool inférieur, comme l'éthanol ou l'isopropanol, et présente une valeur de pH comprise entre 6 et 8.

7. Agent selon les revendications 1 à 5, caractérisé en ce qu'il contient additionnellement un agent tensio-actif cationique, non-ionique, amphotère ou anionique et qu'il se présente sous forme de shampooing.

8. Agent selon la revendication 7, caractérisé en ce que l'agent tensio-actif anionique est un alcoyl-éther-sulfat.

9. Agent selon les revendications 7 et 8, caractérisé en ce qu'il contient l'agent tensio-actif à raison de 3 à 25% en poids et présente une valeur de pH comprise entre 4 et 7.

10. Agent selon les revendications 1 à 6, caractérisé en ce qu'il contient comme base cosmétique une solution alcoolique ou hydro-alcoolique qui est mélangée à un gaz carburant usuel, chargée dans un récipient sous pression et se présente sous la forme d'une pulvérisation d'aérosol.

11. Agent selon les revendications 1 à 3, caractérisé en ce que la base cosmétique est une solution aqueuse, alcoolique ou hydro-alcoolique, une crème, un gel ou une emulsion, qui contient le dérivé de chitosane de formule (I) à raison de 0,1 à 10% en poids et se présente sous la forme d'un agent cosmétique pour le traitement de la peau.

12. Agent selon les revendications 1 à 10, caractérisé en ce qu'il contient additionnellement un polymère cosmétique usuel filmogène naturel ou synthétique.